(19) **Europäisches Patentamt**
**European Patent Office**
**Office européen des brevets**

(11) **EP 4 166 942 A1**

(12) **EUROPEAN PATENT APPLICATION**

(43) Date of publication:
**19.04.2023 Bulletin 2023/16**

(21) Application number: **21202164.6**

(22) Date of filing: **12.10.2021**

(51) International Patent Classification (IPC):
**G01N 33/00** (2006.01) **G01N 27/12** (2006.01)

(52) Cooperative Patent Classification (CPC):
**G01N 27/12; G01N 33/0034**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Infineon Technologies AG**
**85579 Neubiberg (DE)**

(72) Inventors:
• **Carbonelli, Cecilia**
**81477 München (DE)**
• **Mittermaier, Simon**
**85659 Forstern (DE)**
• **Schober, Sebastian**
**80469 München (DE)**
• **Zhao, Jianyu**
**81829 München (DE)**

(74) Representative: **Stöckeler, Ferdinand et al**
**Schoppe, Zimmermann, Stöckeler**
**Zinkler, Schenk & Partner mbB**
**Patentanwälte**
**Radlkoferstrasse 2**
**81373 München (DE)**

(54) **GAS SENSING DEVICE AND METHOD FOR SENSING ONE OR MORE GASES IN A MIXTURE OF GASES**

(57)     The gas sensing device comprises: one or more chemo-resistive gas sensors; one or more heat sources; a preprocessing processor; a feature extraction processor; a discriminative embedding network processor configured for receiving the sets of feature values and for creating for each of the sets of feature values a set of embedded feature values, wherein the discriminative embedding network processor comprises a first trained model based algorithm processor and a first trained model for the first trained model based algorithm processor, wherein the first trained model is configured for applying a loss function using discriminate weights to the sets of feature values in order to create the sets of embedded features values; a classification processor configured for receiving the sets of embedded feature values and for creating a classification value for each set of the embedded feature values, wherein the classification value indicates a class of the mixture of gases, wherein the classification processor comprises a second trained model based algorithm processor and a second trained model for the second trained model based algorithm processor, wherein the sets of embedded feature values are fed to an input of the second trained model based algorithm processor, wherein the classification values are provided at an output of the second trained model based algorithm processor; and a quantification processor configured for receiving the sets of embedded feature values and the classification values, wherein the quantification processor is configured for creating for each of the gases a sensing result for each of the sets of embedded feature values, wherein the quantification processor comprises a third trained model based algorithm processor and a plurality of third trained models for the third trained model based algorithm processor, wherein the sets of embedded feature values are fed to an input of the third trained model based algorithm processor, wherein the sensing result are provided at an output of the third trained model based algorithm processor, wherein one third trained model of the plurality of third trained models is selected for creating the sensing results based on the classification values.

Figure 1

**Description**

Technical Field

Embodiments relate to a gas sensing device for sensing one or more gases in a mixture of gases. Further embodiments relate to a method for operating such gas sensing device. More particular, the disclosure deals with the estimation of gas concentrations through the use of chemo-resistive gas sensors.

Background of the Invention

Literature on chemo-resistive gas sensors is generally limited to a simple model for proof of sensor functionality or costly data acquisition methodologies using geographically distributed sensor systems with impractical implementations [1, 2]. In order to distinguish between different gases, the use of selective physical gas filters or additional non-chemo-resistive gas sensors has been proposed. However, such use has a significant impact on the product sizes and cost [3].

Summary of the Invention

A gas sensing device for sensing one or more gases in a mixture of gases is provided. The gas sensing device comprises:

one or more chemo-resistive gas sensors, wherein each of the gas sensors is configured for generating signal samples corresponding to concentrations of the one or more gases in the mixture of gases;

one or more heat sources, wherein the one or more heat sources are controlled in such way that the gas sensors are each heated according to one or more temperature profiles;

a preprocessing processor configured for receiving the signal samples from each of the gas sensors and for preprocessing the received signal samples in order to generate preprocessed signal samples for each of the gas sensors;

a feature extraction processor configured for receiving the preprocessed signal samples and for extracting a set of feature values from each of the received preprocessed signal samples of the gas sensors based on characteristics of the received preprocessed signal samples of the gas sensors;

a discriminative embedding network processor configured for receiving the sets of feature values and for creating for each of the sets of feature values a set of embedded feature values, wherein the discriminative embedding network processor comprises a first trained model based algorithm processor and a first trained model for the first trained model based algorithm processor, wherein the first trained model is configured for applying a loss function using discriminate weights to the sets of feature values in order to create the sets of embedded features values;

a classification processor configured for receiving the sets of embedded feature values and for creating a classification value for each set of the embedded feature values, wherein the classification value indicates a class of the mixture of gases, wherein the classification processor comprises a second trained model based algorithm processor and a second trained model for the second trained model based algorithm processor, wherein the sets of embedded feature values are fed to an input of the second trained model based algorithm processor, wherein the classification values are provided at an output of the second trained model based algorithm processor; and

a quantification processor configured for receiving the sets of embedded feature values and the classification values, wherein the quantification processor is configured for creating for each of the gases a sensing result for each of the sets of embedded feature values, wherein the quantification processor comprises a third trained model based algorithm processor and a plurality of third trained models for the third trained model based algorithm processor, wherein the sets of embedded feature values are fed to an input of the third trained model based algorithm processor, wherein the sensing result are provided at an output of the third trained model based algorithm processor, wherein one third trained model of the plurality of third trained models is selected for creating the sensing results based on the classification values.

The one or more chemo-resistive gas sensors may be graphene gas sensors or reduced graphene gas sensors, where the base material is functionalized with specific chemicals, e.g. with platinum (Pt), or manganese dioxide (MnO2), so that each of the gas sensors is sensitive for gases, e.g. for nitrogen dioxide (NO2), ozone (O3) or carbon monoxide (CO). In doing so, the interaction between graphene sheets and absorbed gas analytes influences the electronic structure of the material depending on the mixture of gases, resulting in altered charge carrier concentration and changed electrical conductance.

In case of multi-gas sensing a multi-gas sensor array comprising a plurality of chemo-resistive gas sensors having dissimilar selectivity may be used. Due to the different sensitivity towards various gas molecules, resistances of the gas sensors change in disparate patterns, making it possible to analyze complicated gas mixtures with one single sensor array.

A signal sample is a sequence consisting of time-discrete signal values, wherein the signal values are output by one of the gas sensors.

Each of the gas sensors may be heated by one or more heat sources. The heat sources may be electrically powered resistive heating elements or radiators emitting light, in particular with ultra violet light. Each of the one or more heat sources is controlled according to one or more temperature profiles during operational phases. Each of the temperature profiles modulates a temperature of one or more of the gas sensors between a maximum temperature and a minimum temperature.

For example, according to one of the temperature profiles the temperature of the one or more heating elements may be pulsed between a maximum temperature and a minimum temperature The maximum temperature may be, for example, set to a value between 150°C and 300°C, whereas the minimum temperature may be, for example, set to a value between 50°C and 200°C.

In other embodiments, other temperature profiles such as ramps may be used.

The temperature modulation could be the same for all sensors or different for at least some of the sensors.

The temperature modulation improves repeatability and stability of the sensing results.

The term processor refers to an electronic device configured for specific task. A processor may comprise hardware or a combination of hardware and software. Different processors may share hardware components and/or software components.

The pre-processing processor is configured for suppressing and/or compensating of artifacts in the signal samples and/or noise in the signal samples and/or invalid signal samples due to malfunctioning gas sensors and/or errors in the signal samples due to drifts of the gas sensors in order to produce more reliable filtered signal samples.

The feature extraction processor is configured for receiving the preprocessed signal samples and for extracting one or more feature values from the received preprocessed signal samples of each of the gas sensors based on characteristics of the received preprocessed signal samples of the respective gas sensor. The features may be based on dynamic characteristics of the signal samples. To this end, the modulated nature of the responses of the gas sensors is leveraged and characteristics are extracted which rely on the dynamic evolution of the gas sensors.

The core of the invention is a gas sensing device having an improved detection mechanism, where a discriminative embedding network processor is used followed by a classification processor and a quantification processor so as to reduce cross-sensitivity and to improve estimation accuracy for each single target gas in a mixture of gases.

A trained model based algorithm processor is a processor, which is capable of machine learning. The machine learning is done in a preoperational training phase in which trained models are developed by comparing actual output values of the trained model based algorithm stage with desired output values of the trained model based algorithm stage for defined inputs of the trained model based algorithm stage. The trained models have a predefined structure, wherein a parametrization of the predefined structure is done during the training phase. The trained models comprise the learned content after the training phase is finished. In an operational phase for producing processing results one or more of the trained models from the training phase are used to process their input data.

In the training phase, the plurality of trained models can be established and afterwards stored at the gas sensing device. The trained models may differ in the structures and/or the parameters. During the operation phase the most appropriate trained model may be selected depending on the specific use-case.

The discriminative embedding network processor is configured for embedding the sets of feature values into a new space so that the sets of embedded feature values have better separability. In other words, the discriminative embedding network processor applies a discriminative mapping to the sets of feature values in order to generate sets of embedded feature values so that embedded feature values belonging to the same gas or gas mixtures are closer to each other and those belonging to different gases or gas mixtures are further apart.

The classification processor is configured for creating a classification value for each set of the embedded feature values, wherein the classification value indicates a class of the mixture of gases. In other words, the classification processor predicts for each of the sets of embedded feature values a class or a usecase and recommends a regression mode to be used by the quantification processor for the quantification of the gas concentration(s).

The quantification processor is configured for producing sensing results depending on the sets of embedded feature values and depending on the classification value. In other words, the quantification processor is a regressor which applies the recommended regression mode to the sets of embedded values in order to obtain one or multiple gas concentration estimates.

The sensing results may be alphanumeric terms, for example alphanumeric terms on a scale from "high" to "low". In particular, the terms of an air quality index system, for example terms of the European air quality index as described in [4], may be used for outputting the sensing results. In other embodiments, the sensing results may be physical quantities such as "4% by volume".

The combination of the discriminative embedding network processor, the classification processor and the quantification processor improves the accuracy of the sensing results. As the quantification is performed depending on the class of the mixture of gases, this is valid for different mixtures of gases. Furthermore, the gas sensor may be adapted for the detection of additional gases without changing the hardware, as it is sufficient to modify the third trained models of the quantification processor. In addition, it is possible to adapt the gas sensing device to additional mixtures of gases, as it is sufficient to modify the second trained model of the classification processor.

The proposed gas sensing device provides an end to end solution for multi-gas adsorption sensors which is versatile, widely-applicable to multiple applications and uses cases (indoor or outdoor air quality monitoring, health checks, such as breath analysis or disease diagnostics, etc.) and can be embedded in a smart portable device.

According to embodiments of the disclosure the one or more gas sensors are alternately operated in recovery phases and in sense phases;

wherein the one or more heat sources are controlled in such way that the gas sensors are each heated according to one or more first temperature profiles of the one or more temperature profiles during the recovery phases and according to one or more second temperature profiles of the one or more temperature profiles during the sense phases, wherein for each of the gas sensors a maximum temperature of the respective first temperature profile is higher than a maximum temperature of the respective second temperature profile.

Each of the one or more heat sources may be controlled according to one or more temperature profiles during the recovery phases and according to a second temperature profile during the sense phases, wherein a maximum temperature of the first temperature profile is higher than a maximum temperature of the second temperature profile.

For example, the temperature of the one or more heating elements may be pulsed between a first temperature during the recovery phases of the gas sensors and a second temperature during the sense phases of the gas sensors, wherein the first temperature is higher than the second temperature. The first temperature may be, for example, set to a value between 150°C and 300°C, whereas the second temperature may be, for example, set to a value between 50°C and 200°C.

The temperature modulation could be the same for all sensors or different for at least some of the sensors.

In order to improve repeatability and stability of the sensing results, at least some of the signal samples of each of the gas sensors may represent at least one of the recovery phases and at least one of the sense phases.

According to embodiments of the disclosure a number of the chemo-resistive gas sensors is greater than one, wherein at least some of the chemo-resistive gas sensors have different sensitivities towards one or more of the gases. Such features further improve the accuracy of the sensing results.

According to embodiments of the disclosure the preprocessing processor is configured for executing a baseline calibration algorithm for the signal samples received from the gas sensors. Baseline manipulation is the transformation of a signal sample of one of the gas sensors into a relative resistance change with respect to sensor response to a reference analyte, wherein such sensor response is called a baseline. Synthetic air is a very common baseline as it is easily applicable and realistic in a real world scenario. The purpose of a baseline is to potentially create a more stable and reproducible sensing result by removing some of the drift caused by long term gas exposure and ageing of the sensor. As shown in Equation (1), subtracting the sensor response by its baseline $R_0$ removes additive drift while division removes multiplicative drift. Using both operations combined results in the relative resistance change $\Delta R/R_0$:

$$\Delta R/R_0 = (R - R_0)/R_0 \qquad (1)$$

According to embodiments of the disclosure the preprocessing processor is configured for executing a filtering algorithm for the signal samples received from the gas sensors. The filtering algorithm may, for example, be implemented as a high pass filter or a noise filter. Such features further improve the accuracy of the sensing results.

According to embodiments of the disclosure the feature extraction processor is configured for extracting from the received preprocessed signal samples a normalized sensor sensitivity $\Delta R/R_0$ as one of the feature values for each of the gas sensors. The normalized sensor sensitivity $\Delta R/R_0$ may be calculated according to Equation (1).

Using the normalized sensor sensitivities $\Delta R/R_0$ as feature values improves the accuracy of the sensing results.

According to embodiments of the disclosure the feature extraction processor is configured for extracting from the received preprocessed signal samples a slope $R'(t)$ of one of the preprocessed signal samples as one of the feature values for each of the gas sensors. The slope $R'(t)$ or derivative may be calculated according to Equation (2):

$$R'(t) = \Delta R(t) / \Delta t \qquad (2)$$

Using the slopes $R'(t)$ as feature values improves the accuracy of the sensing result.

According to embodiments of the disclosure the feature extraction processor is configured for extracting from the received preprocessed signal samples for each of the gas sensors a time correlation between a first of the preprocessed signal samples of the respective gas sensor and a second preprocessed signal sample of the respective gas sensor as one of the feature values for the respective gas sensor.

According to embodiments of the disclosure the feature extraction processor is configured for extracting from the received preprocessed signal samples for each of the gas sensors a spatial correlation between one of the preprocessed signal samples of the respective gas sensor and one of the preprocessed signal sample of another of the gas sensors as one of the feature values for the respective gas sensor.

Given the dynamic behavior of the gas sensors, the availability of several transient in the sensor responses and the characteristic array structure with different functionalizations, it makes sense to introduce metrics which exploits such time and spatial properties. This can be achieved introducing a time autocorrelation function of the normalized sensor responses of the type (and its derivative)

$$R_\tau = \sum_{k=1}^{n} x_k y_k \qquad (3)$$

Where $x$ and $y$ indicate the normalized response at different moments in time (or, alternatively, their derivatives) and $n$ is the window size being used to calculate the autocorrelation. Particularly:

$$x_k = \frac{\Delta R(k)}{R_0}; \; x_k = \Delta R(k + \tau)/R_0 \qquad (4)$$

Similarly, the correlation among the different gas sensors should also be exploited with a spatial correlation matrix of the type:

$$R_s[r,p] = \frac{1}{n}\sum_{i=1}^{n} x_{i,r} x_{i,p} \qquad (5)$$

According to embodiments of the disclosure the first trained model based algorithm processor is implemented as a first artificial neural network.

According to embodiments of the disclosure the second trained model based algorithm processor is implemented as a second artificial neural network, in particular as a fully connected artificial neural network.

According to embodiments of the disclosure the third trained model based algorithm processor is implemented as a third artificial neural network, in particular as a second gated recurrent unit followed by a fully connected artificial neural network.

An artificial neural network is a parameterized statistic model, in which a number of logistic regressions are combined non-linearly. Such systems "learn" to perform tasks by considering examples, generally without being programmed with any task-specific rules. A neural network is based on a collection of connected nodes called artificial neurons. Each connection can transmit a signal from one artificial neuron to another. An artificial neuron that receives a signal can process it and then signal additional artificial neurons connected to it. A model predefines the structure of the nodes or the hyperparameters of a neural network and the parameters of the connections are found by training the neural network. Structure and the corresponding parameters form a trained model for the respective neural network.

A fully connected artificial neural network is an artificial neural network in which every neuron in one layer is connected to every neuron in the next layer. A gated recurrent unit is recurrent neural network using gating mechanisms.

According to embodiments of the disclosure the discriminative embedding network processor comprises a plurality of first gated recurrent units and a discriminative loss computation processor, which are configured for optimizing parameters, in particular weights and/or offsets, of the first trained model.

According to embodiments of the disclosure the third trained model based algorithm processor is implemented as a third artificial neural network, in particular as a second gated recurrent unit followed by a fully connected artificial neural network.

According to embodiments of the disclosure the classification processor is configured for preventing the quantification processor from creating sensing results, in case the classification processor is unable to create one of the classification value for one of the sets of the embedded feature values.

In a further aspect of the disclosure, a method for operating a gas sensing device for sensing one or more gases in a mixture of gases, which comprises one or more chemo-resistive gas sensors is disclosed. The method comprises the steps of:

using each of the gas sensors for generating signal samples corresponding to concentrations of the one or more gases in the mixture of gases;

using one or more heat sources for heating each of the gas sensors according to one or more temperature profiles;

using a preprocessing processor for receiving the signal samples from each of the gas sensors and for preprocessing the received signal samples in order to generate preprocessed signal samples for each of the gas sensors;

using a feature extraction processor for receiving the preprocessed signal samples and for extracting one or more feature values from the received preprocessed signal samples of each of the gas sensors based on characteristics of the received preprocessed signal samples of the respective gas sensor;

using a discriminative embedding network processor for receiving the sets of feature values and for creating for each of the sets of feature values a set of embedded feature values, wherein the discriminative embedding network processor comprises a first trained model based algorithm processor and a first trained model for the first trained model based algorithm processor, wherein the first trained model is configured for applying a loss function using discriminate weights to the sets of feature values in order to create the sets of embedded features values;

using a classification processor for receiving the sets of embedded feature values and for creating a classification value for each set of the embedded feature values, wherein the classification value indicates a class of the mixture of gases, wherein the classification processor comprises a second trained model based algorithm processor and a second trained model for the second trained model based algorithm processor, wherein the sets of embedded feature values are fed to an input of the second trained model based algorithm processor, wherein the classification values are provided at an output of the second trained model based algorithm processor; and

using a quantification processor configured for receiving the sets of embedded feature values, for receiving the classification values, and for creating for each of the gases a sensing result for each of the sets of embedded feature values, wherein the quantification processor comprises a third trained model based algorithm processor and a plurality of third trained models for the third trained model based algorithm processor, wherein the sets of embedded feature values are fed to an input of the third trained model based algorithm processor, wherein the sensing result are provided at an output of the third trained model based algorithm processor, wherein one third trained model of the plurality of third trained models is selected for creating the sensing results based on the classification values.

Preferred embodiments of the invention are subsequently discussed with respect to the accompanying drawings, in which:

Figure 1    shows a schematic view of an exemplary embodiment of a gas sensing device according to the disclosure, which comprises four chemo-resistive gas sensors;

Figure 2    shows a more specific schematic view of an exemplary embodiment of a gas sensing device according to the disclosure, which comprises four chemo-resistive gas sensors;

Figure 3    shows a schematic view of an exemplary embodiment of a discriminative embedding network processor according to the disclosure;

Figure 4    shows a schematic view of an exemplary embodiment of a classification processor according to the disclosure;

Figure 5    shows a schematic view of an exemplary embodiment of a quantification processor according to the disclosure;

Figure 6    shows a scatter plot of a linear discriminant analysis of exemplary feature values provided by the feature extraction processor;

Figure 7     shows a scatter plot of a linear discriminant analysis of the output of the second gated recurrent unit in case that the discriminative embedding network processor is deactivated;

Figure 8     shows a scatter plot of a linear discriminant analysis of the output of the second gated recurrent unit in case that the discriminative embedding network processor is activated;

Figure 9     shows exemplary sensing results for nitrogen dioxide and ozone over time as well as the classification value over time;

Figure 10     shows an exemplary graphene multi-gas sensor array according to the disclosure; and

Figure 11     illustrates exemplary normalized sensor responses and heater temperatures over time.

Equal or equivalent elements or elements with equal or equivalent functionality are denoted in the following description by equal or equivalent reference numerals.

In the following description, a plurality of details is set forth to provide a more thorough explanation of embodiments of the present disclosure. However, it will be apparent to those skilled in the art that embodiments of the present disclosure may be practiced without these specific details. In other instances, well-known structures and devices are shown in block diagram form rather than in detail in order to avoid obscuring embodiments of the present disclosure. In addition, features of the different embodiments described hereinafter may be combined with each other, unless specifically noted otherwise.

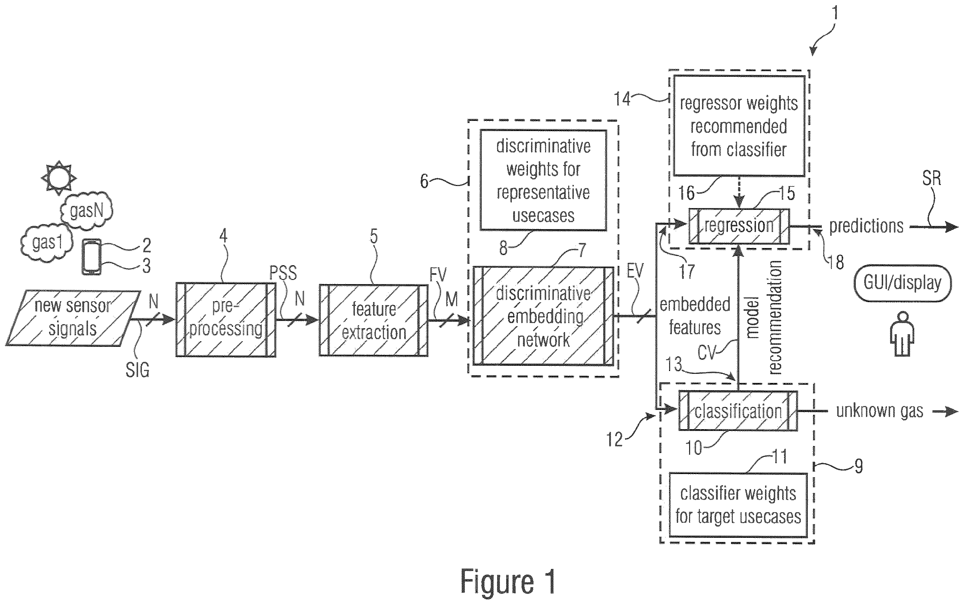

Figure 1 shows a schematic view of an exemplary embodiment of a gas sensing device 1 according to the disclosure, which comprises four chemo-resistive gas sensors 2.

The gas sensing device 1 is configured for sensing one or more gases in a mixture of gases. The gas sensing device 1 comprises:

one or more chemo-resistive gas sensors 2, wherein each of the gas sensors 2 is configured for generating signal samples SIG corresponding to concentrations of the one or more gases in the mixture of gases;

one or more heat sources 3, wherein the one or more heat sources 3 are controlled in such way that the gas sensors 2 are each heated according to one or more temperature profiles FTP, STP;

a preprocessing processor 4 configured for receiving the signal samples SIG from each of the gas sensors 2 and for preprocessing the received signal samples SIG in order to generate preprocessed signal samples PSS for each of the gas sensors 2;

a feature extraction processor 5 configured for receiving the preprocessed signal samples PSS and for extracting a set of feature values FV from each of the received preprocessed signal samples PSS of the gas sensors 2 based on characteristics of the received preprocessed signal samples PSS of the gas sensors 2;

a discriminative embedding network processor 6 configured for receiving the sets of feature values FV and for creating for each of the sets of feature values FV a set of embedded feature values EV, wherein the discriminative embedding network processor 6 comprises a first trained model based algorithm processor 7 and a first trained model 8 for the first trained model based algorithm processor 7, wherein the first trained model 8 is configured for applying a loss function using discriminate weights to the sets of feature values FV in order to create the sets of embedded features values EV;

a classification processor 9 configured for receiving the sets of embedded feature values EV and for creating a classification value CV for each set of the embedded feature values EV, wherein the classification value CV indicates a class of the mixture of gases, wherein the classification processor 9 comprises a second trained model based algorithm processor 10 and a second trained model 11 for the second trained model based algorithm processor 10, wherein the sets of embedded feature values EV are fed to an input 12 of the second trained model based algorithm processor 10, wherein the classification values CV are provided at an output 13 of the second trained model based algorithm processor 10; and

a quantification processor 14 configured for receiving the sets of embedded feature values EV and the classification values CV, wherein the quantification processor 14 is configured for creating for each of the gases a sensing result SR for each of the sets of embedded feature values EV, wherein the quantification processor 14 comprises a third trained model based algorithm processor 15 and a plurality of third trained models 16 for the third trained model based algorithm processor 15, wherein the sets of embedded feature values EV are fed to an input 17 of the third trained model based algorithm processor 15, wherein the sensing result SR are provided at an output 18 of the third trained model based algorithm processor 15, wherein one third trained model 16 of the plurality of third trained models 16 is selected for creating the sensing results SR based on the classification values CV.

According to embodiments of the disclosure the preprocessing processor 4 is configured for executing a baseline calibration algorithm for the signal samples SIG received from the gas sensors 2.

According to embodiments of the disclosure the preprocessing processor 4 is configured for executing a filtering algorithm for the signal samples SIG received from the gas sensors 2.

According to embodiments of the disclosure the feature extraction processor 5 is configured for extracting from the received preprocessed signal samples PSS a normalized sensor sensitivity as one of the feature values FV for each of the gas sensors 2.

According to embodiments of the disclosure the feature extraction processor 5 is configured for extracting from the received preprocessed signal samples PSS a slope of one of the preprocessed signal samples PSS as one of the feature values FV for each of the gas sensors 2.

According to embodiments of the disclosure the feature extraction processor 5 is configured for extracting from the received preprocessed signal samples PSS for each of the gas sensors 2 a time correlation between a first of the preprocessed signal samples PSS of the respective gas sensor 2 and a second preprocessed signal sample PSS of the respective gas sensor 2 as one of the feature values FV for the respective gas sensor 2.

According to embodiments of the disclosure the feature extraction processor 5 is configured for extracting from the received preprocessed signal samples PSS for each of the gas sensors 2 a spatial correlation between one of the preprocessed signal samples PSS of the respective gas sensor 2 and one of the preprocessed signal sample PSS of another of the gas sensors 2 as one of the feature values FV for the respective gas sensor 2.

According to embodiments of the disclosure the classification processor 9 is configured for preventing the quantification processor 14 from creating sensing results, in case the classification processor 9 is unable to create one of the classification value CV for one of the sets of the embedded feature values EV.

In a further aspect, the disclosure refers to a method for operating a gas sensing device 1 for sensing one or more gases in a mixture of gases, wherein the gas sensing device 1 comprises one or more chemo-resistive gas sensors 2, wherein the method comprises the steps of:

using each of the gas sensors 2 for generating signal samples SIG corresponding to concentrations of the one or more gases in the mixture of gases;

using one or more heat sources 3 for heating each of the gas sensors 2 according to one or more temperature profiles FTP, STP;

using a preprocessing processor 4 for receiving the signal samples SIG from each of the gas sensors 2 and for preprocessing the received signal samples SIG in order to generate preprocessed signal samples PSS for each of the gas sensors 2;

using a feature extraction processor 5 for receiving the preprocessed signal samples PSS and for extracting one or more feature values FV from the received preprocessed signal samples PSS of each of the gas sensors 2 based on characteristics of the received preprocessed signal samples PSS of the respective gas sensor 2;

using a discriminative embedding network processor 6 for receiving the sets of feature values FV and for creating for each of the sets of feature values FV a set of embedded feature values EV, wherein the discriminative embedding network processor 6 comprises a first trained model based algorithm processor 7 and a first trained model 8 for the first trained model based algorithm processor 7, wherein the first trained model 8 is configured for applying a loss function using discriminate weights to the sets of feature values FV in order to create the sets of embedded features values EV;

using a classification processor 9 for receiving the sets of embedded feature values EV and for creating a classification value CV for each set of the embedded feature values EV, wherein the classification value CV indicates a class of the mixture of gases, wherein the classification processor 9 comprises a second trained model based algorithm processor 10 and a second trained model 11 for the second trained model based algorithm processor 10, wherein the sets of embedded feature values EV are fed to an input 12 of the second trained model based algorithm processor 10, wherein the classification values CV are provided at an output 13 of the second trained model based algorithm processor 10; and

using a quantification processor 14 configured for receiving the sets of embedded feature values EV, for receiving the classification values CV, and for creating for each of the gases a sensing result SR for each of the sets of embedded feature values EV, wherein the quantification processor 14 comprises a third trained model based algorithm processor 15 and a plurality of third trained models 16 for the third trained model based algorithm processor 15, wherein the sets of embedded feature values EV are fed to an input 17 of the third trained model based algorithm processor 15, wherein the sensing result SR are provided at an output 18 of the third trained model based algorithm processor 15, wherein one third trained model 16 of the plurality of third trained models 16 is selected for creating the sensing results SR based on the classification values CV.

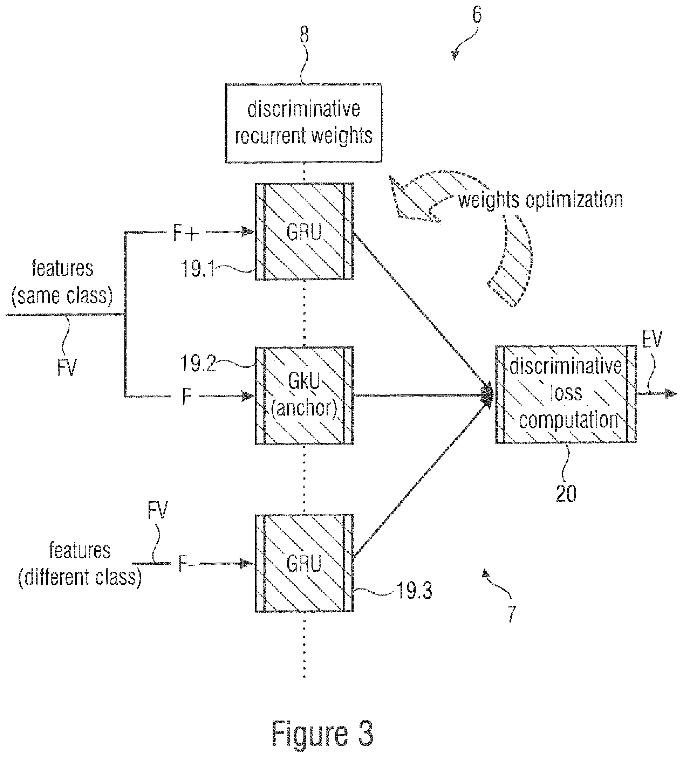

After the preprocessing processor 4 and feature extraction processor 5 a discriminative embedding processor 6 is introduced which embeds the sets of feature values FV into a new space where the embedded feature values EV have better separability. The discriminative embedding processor 6 may be implemented as a neural network with a contrastive 'distance-learning' loss function such that, after transformation, embedded feature values EV belonging to the same gas or gas mixture types are closer to each other and those belonging to different gas types or gas mixtures are further apart. An example is provided in Figure 3.

It is sufficient to train the discriminative embedding network processor 6 on a selection of representative mixtures of gases. Afterwards, the discriminative embedding network processor 6 shall be capable of discriminating also unseen gases or use-cases.

Depending on the target application of the product, certain target gases or mixtures of gases have to be identified. For example, it was observed that, in a mixture, nitrogen dioxide is masked by ozone and, as such, nitrogen dioxide cannot - and shall not - be estimated in the presence of ozone. In this specific case, we need to identify three classes: specifically, nitrogen dioxide only, air, or ozone dominated gas mixture. Thresholds may be used to identify the different categories. For instance, 'nitrogen dioxide only' means nitrogen dioxide >1 ppb and ozone < 10ppb, 'ozone - mixture' corresponds to ozone >=10 ppb and 'Air' means ozone <10ppb and nitrogen dioxide <1 ppb, and so on.

For other products/applications, a mixture of gases can still come into play (for example a mixture of ozone and carbon monoxide) and, as such, this class and respective weights will have to be enabled at the classification processor 9.

Thanks to the initial discriminative feature embedding, the classification processor 9 can quite reliably identify, for each set of embedded feature values EV over time, the relevant class or scenario and, as such, recommend a certain third trained model 16 (regressor model) to the quantification processor 14, which eventually quantifies the concentration of the gases of interest for the corresponding time sample.

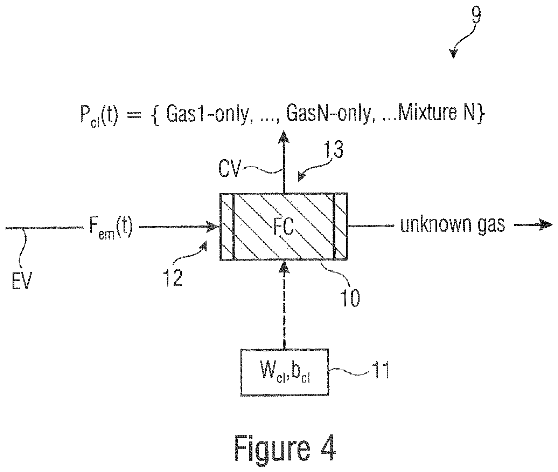

Furthermore, the classification processor 9 can also be equipped with an additional output, which detects an unknown gas for which then no concentration shall be estimated since no third trained model 16 is available for it. A possible implementation is shown in Figure 4.

The quantification processor 14 may apply O3-mixture and NO2-only weights from a specific third trained model 16 to the incoming sets of embedded feature values EV. In other applications, where the gas sensing device 1 is selective to more gases and more than one gas concentration has to be estimated at the same time, then gas mixture weights from another third trained model 16 will be applied.

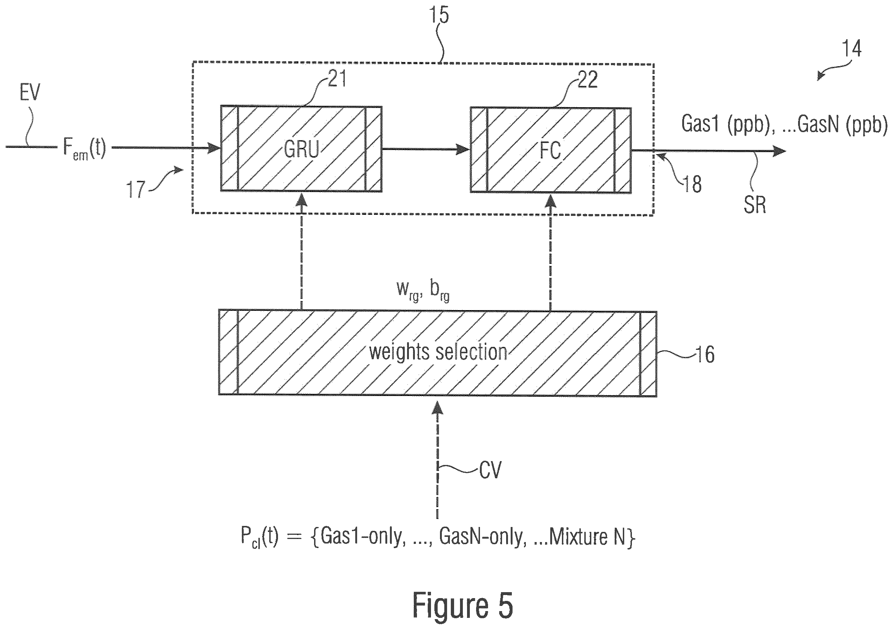

Thanks to the initial discriminative feature embedding and to the presence of the classification processor 9, which provides the quantification processor 14 with additional information on the specific scenario, the quantification processor 14 can be greatly simplified and one can resort to simpler processing steps. A possible implementation is shown in Figure 5.

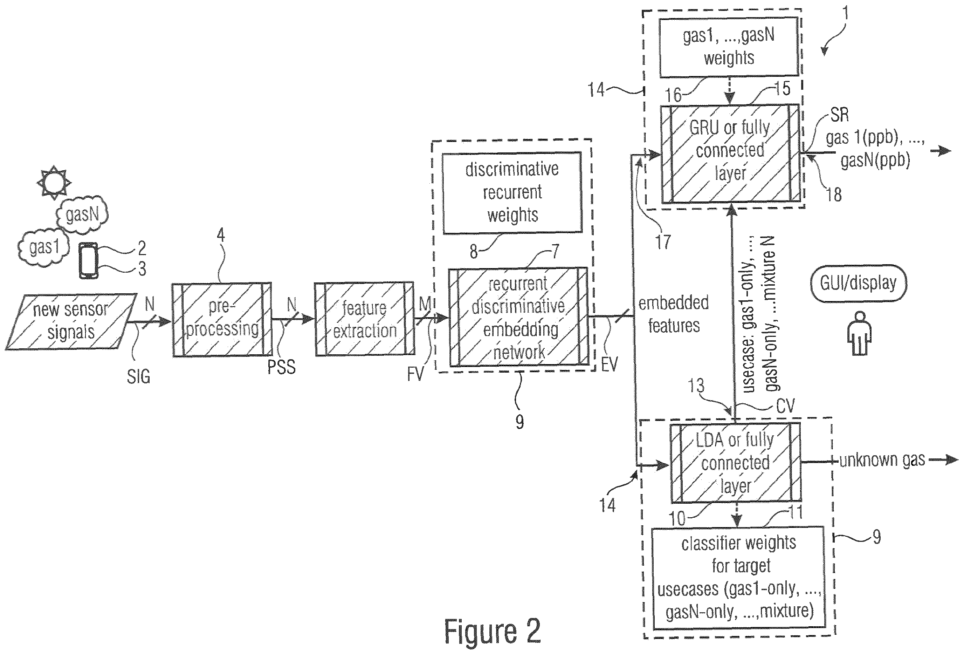

Figure 2 shows a more specific schematic view of an exemplary embodiment of a gas sensing device 1 according to the disclosure, which comprises four chemo-resistive gas sensors 2.

Figure 3 shows a schematic view of an exemplary embodiment of a discriminative embedding network processor 6 according to the disclosure.

According to embodiments of the disclosure the first trained model based algorithm processor 6 is implemented as a first artificial neural network.

According to embodiments of the disclosure the discriminative embedding network processor 6 comprises a plurality of first gated recurrent units 19 and a discriminative loss computation processor 20, which are configured for optimizing parameters, in particular weights and/or offsets, of the first trained model 8.

The discriminative embedding network 6 may be implemented as a triplet gated recurrent unit network with triplet loss defined as

$$Loss = max(D_+ - D_- + \alpha, 0.0)$$

where

$$D_+ = \sum (GRU(F) - GRU(F^+))^2$$

$$D_- = \sum (GRU(F) - GRU(F^-))^2$$

and $\alpha$ is a parameter such that $0 < \alpha < 1$ . The training strategy is shown in Figure 3 where three gated recurrent units 19 are being fed with sets of features values FV from the same or from a different class as the central (anchor) gated recurrent unit 19.2 and all weights are shared. The learned weights are then applied as the first trained model 8 to the first trained model based algorithm processor 7 of the recurrent embedded discriminative network processor 6 in Figure 2.

The recurrent nature of the triplet approach above achieves superior performance when dealing with time series data from low cost gas sensors 2. The difficulty here is that feature values FV from the past need to be included, since instantaneous feature values FV are not sufficient to correctly identify and learn the dynamics of the signal samples SIG.

Furthermore, thanks to the discriminative embedding network processor 6 in Figure 3 and its embedding properties, good gas prediction performance can be achieved with simplified schemes both at the classification processor 9 and at the quantification processor 14.

Figure 4 shows a schematic view of an exemplary embodiment of a classification processor 9 according to the disclosure.

According to embodiments of the disclosure the second trained model based algorithm processor 13 is implemented as a second artificial neural network, in particular as a fully connected artificial neural network.

According to embodiments of the disclosure the second trained model based algorithm processor 10 is implemented as an incremental linear discriminant analysis processor (not shown).

In its simplest form, the classification processor 9 could be implemented as a fully connected (FC) neural network with weights adjusted to the target output of the gas sensing device 1. As illustrated in Figure 4, the values $F_{em}$ (t) of the embedded features values FV are transformed applying the pre-trained weights and offsets, $W_{cl}$ and $b_{cl}$ , and a softmax operator is applied to the output of this transformation to generate an output $P_{cl}$ (t).

$$P_{cl}(t) = softmax(W_{cl}F_{em}(t) + b_{cl})$$

For each temporal set of embedded feature values EV, the classification processor 9 assigns a value $P_{cl}$ (t) of the classification value CV and passes this information to the quantification processor 14 . For example, $P_{cl}$ (t) could be chosen out of the set NO2-only', O3-Mixture' or 'Air'. Alternatively, a linear discriminant analysis classifier could also replace the fully connected layer in Figure 4.

Figure 5 shows a schematic view of an exemplary embodiment of a quantification processor 14 according to the disclosure.

According to embodiments of the disclosure the third trained model based algorithm processor 15 is implemented as a third artificial neural network, in particular as a second gated recurrent unit 21 followed by a fully connected artificial neural network 22.

Based on the recommendation of the classification processor 9, the appropriate pre-trained set of weights and offsets, $W_{rg}$ and $b_{rg}$, may be selected for second gated recurrent unit 21 and the second gated recurrent unit 21, applied to the same values $F_{em}(t)$ of the embedded feature values EV used at the classification processor 9 and one or multiple gas predictions, for example in ppb/ppm, are finally delivered as illustrated in Figure 5.

If an unknown gas is found or if the classification processor 9 is not sure about its own output decision, which can be determined by the differences among class probabilities not exceeding a certain threshold, then the quantification processor 14 output may be put on standby.

Thanks to the improved embedded representation, simpler 1-D (single gas) weights/filters can be applied at the regressor as an alternative to complex 2D weights/filters learnt in gas mixture scenarios.

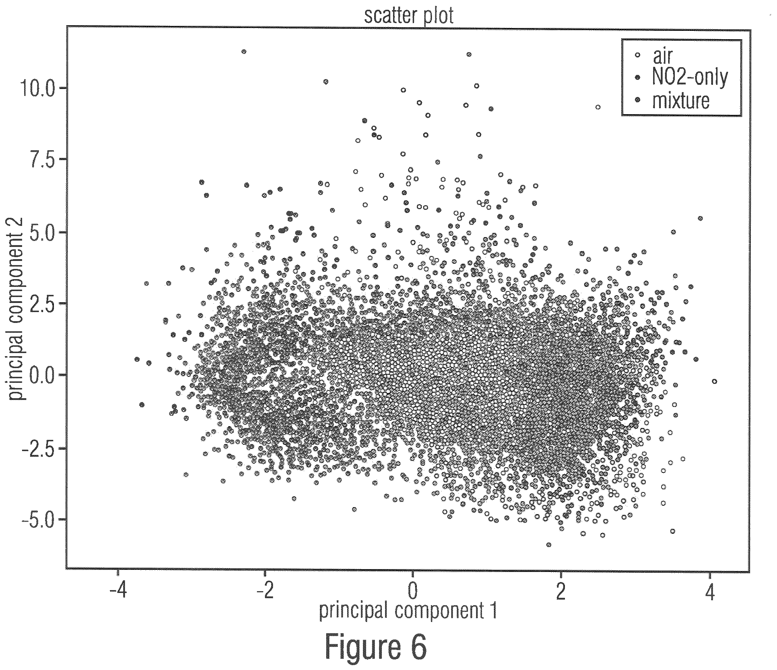

Figure 6 shows a scatter plot of a linear discriminant analysis of exemplary feature values FV provided by the feature extraction processor 5. Each dot illustrates a first principal component and a second principal component of a set of feature values FV. A first group of dots belongs to an air-scenario, a second group of dots belongs to an nitrogen dioxide only scenario and a third group of dots belong to mixture scenario.

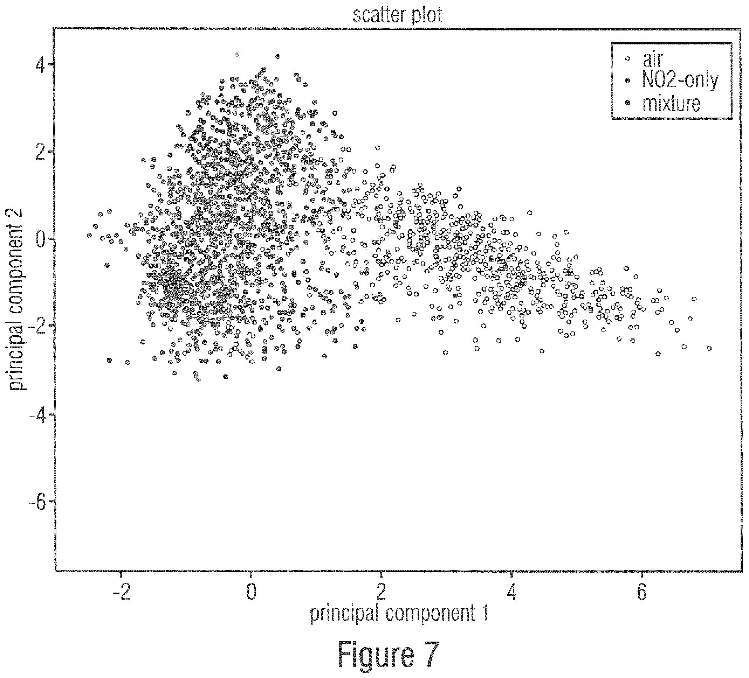

Figure 7 shows a scatter plot of a linear discriminant analysis of the output of the second gated recurrent unit 21 in case that the discriminative embedding network processor 6 is deactivated. Each dot illustrates a first principal component and a second principal component of an output of the second gated recurrent unit, which corresponds to one of the sets of feature values FV.

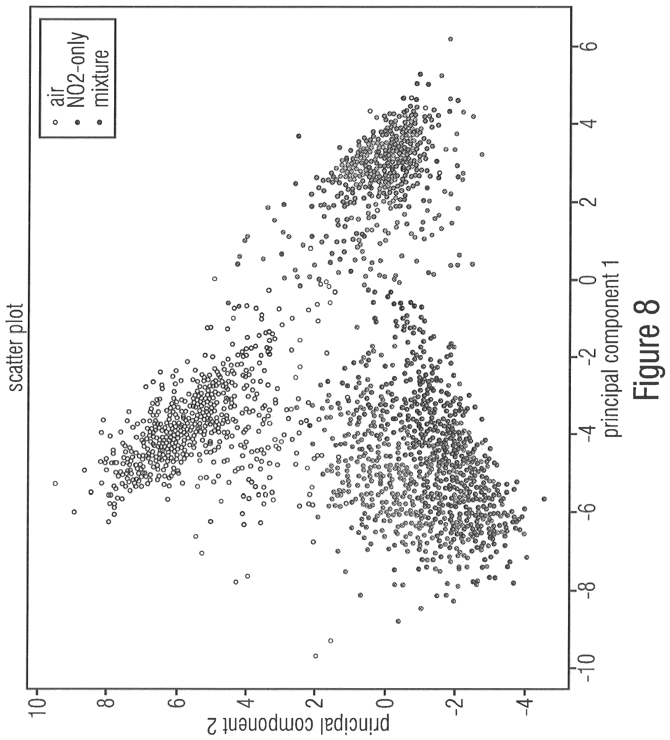

Figure 8 shows a scatter plot of a linear discriminant analysis of the output of the second gated recurrent unit 21 in case that the discriminative embedding network processor 6 is activated. Similarly as in Figure 7, each dot illustrates a first principal component and a second principal component of an output of the second gated recurrent unit, which corresponds to one of the sets of feature values FV. However, dots belonging to the same gas scenario or gas mixture scenario are closer to each other and those belonging to different gas scenarios or gas mixture scenarios are further apart

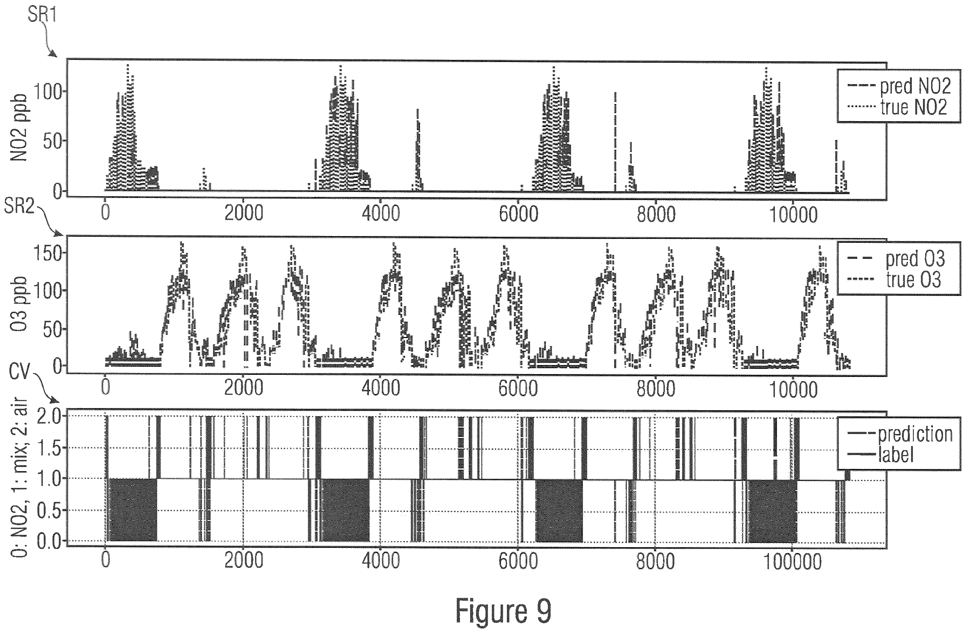

Figure 9 shows exemplary sensing results SR for nitrogen dioxide and ozone over time as well as the classification value CV over time.

The upper chart shows the values "Pred N02" of the sensing results SR1 for a first gas, which is in this example nitrogen oxide, and the true values "True NO2" for the first gas over time.

The chart in the middle shows the values "Pred O3" of the sensing results SR2 for a second gas, which is in this example ozone, and the true values "True O3" for the second gas over time.

The lower chart shows the values "prediction" of the classification values CV and the true values "label" of the scenario over time.

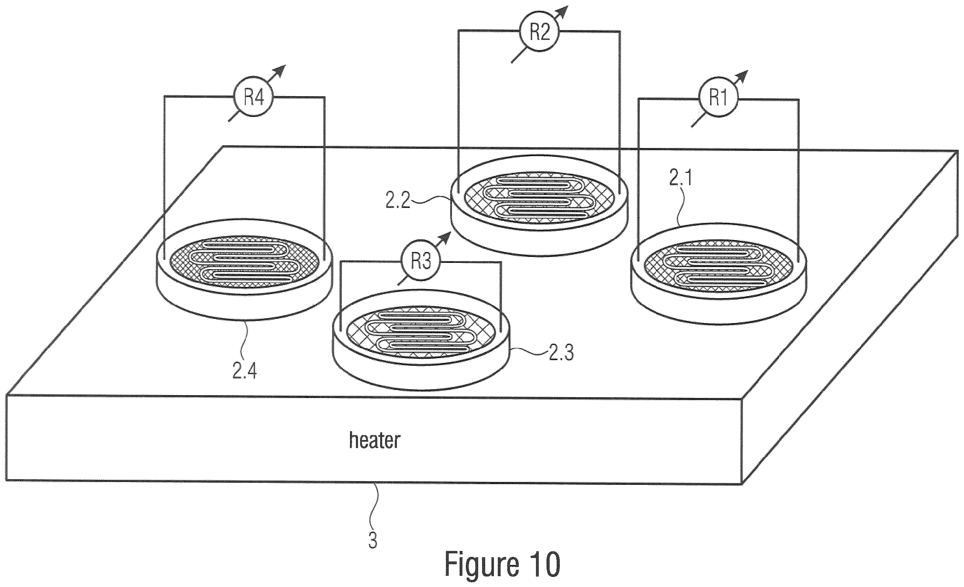

Figure 10 shows an exemplary graphene multi-gas sensor array according to the disclosure.

According to embodiments of the disclosure a number of the chemo-resistive gas sensors 2 is greater than one, wherein at least some of the chemo-resistive gas sensors 2 have different sensitivities towards one or more of the gases.

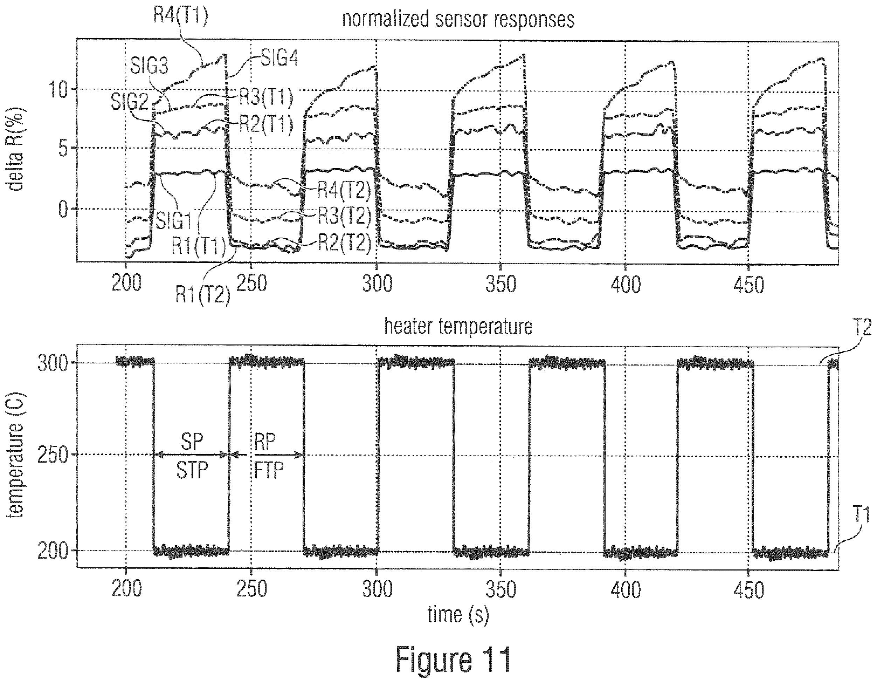

Each sensor 2.1, 2.2, 2.3 and 2.4 in the array is heated by a heat source 3, whose temperature is being pulsed between first temperature T1 during a recovery phase and a second temperature T2 during sense phase (see Figure 11). In other embodiments, the sensors 2.1, 2.2, 2.3 and 2.4 in the array are heated by a plurality of heat sources 3. For example, each of the sensors 2.1, 2.2, 2.3 and 2.4 could be heated individually by one heat source of the plurality of the heat sources. The result of these controlled temperature oscillations is a more dynamic behavior of the signal samples SIG1, SIG2, SIG3, SIG4 as shown in Figure 11, which is exploited by the gas sensing device 1.

Several implementations of temperature pulsing mechanism are possible. For example, the temperature modulation could be the same for all sensors 2.1, 2.2, 2.3 and 2.4 or different in order to better exploit the different functionalizations of the base material and to improve gas separability. Similarly, multiple heater controls can be used (one for each sensor 2.1, 2.2, 2.3 and 2.4) or, alternatively, a single heater control in time division multiplexing with different applied voltages so as to obtain sensor specific temperature values.

The sensors 2.1, 2.2, 2.3 and 2.4 form a multi-gas sensor array, where a base material consisting of graphene is functionalized with different chemicals (e.g. Pd, Pt, and $MnO_2$) for dissimilar selectivity. The interaction between graphene sheets and absorbed gas analytes would influence the electronic structure of the material, resulting in altered charge carrier concentrations and changed electrical conductance. Meanwhile, due to different sensitivity towards various gas molecules resistances of the sensors 2.1, 2.2, 2.3 and 2.4 also change in disparate patterns, making it possible to analyze complicated gas mixtures with one single sensor array.

Figure 11 illustrates exemplary normalized signal samples SIG1, SIG2, SIG3 SIG4 for the chemo-resistive gas sensors 2.1, 2.2, 2.3, 2.4 and temperature profiles FTP, STP over time.

According to embodiments of the disclosure the one or more gas sensors 2 are alternately operated in recovery phases RP and in sense phases SP;

wherein the one or more heat sources 3 are controlled in such way that the gas sensors 2 are each heated according to one or more first temperature profiles FTP of the one or more temperature profiles FTP, STP during the recovery phases RP and according to one or more second temperature profiles STP of the one or more temperature profiles FTP, STP during the sense phases SP, wherein for each of the gas sensors 2 a maximum temperature of the respective first temperature profile FTP is higher than a maximum temperature of the respective second temperature profile STP.

In the particular example of Figure 11 two temperatures profiles FTP, STP are chosen: A first temperature profile FTP for sensing the sensor resistances and for recovering the sensors surface and desorb adsorbed gas molecules at a constant temperature of 300°C in a recovery phase RP and a second temperature profile STP for sensing the sensor resistances at a constant temperature of 200°C during a sense phase SP. Therefore, not only static features like absolute or relative sensor resistance changes can be monitored, but also dynamic features like e.g. the slope of the sense phase SP at 200°C, which reflects the gas adsorption over time. According to Figure 11, the signal samples SIG1, SIG2, SIG3 SIG4 are produced during the sense phases SP and during the recovery phases RP. However, in other embodiments, the signal samples SIG1, SIG2, SIG3 SIG4 may be produced during the sense phases SP only. Additional temperature steps and pulse modes are also possible, as long as they contribute additional information or features to the signal samples SIG1, SIG2, SIG3 and SIG4 like gas adsorption/reaction at a certain temperature or temperature ramp.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

The above described is merely illustrative, and it is understood that modifications and variations of the arrangements and the details described herein will be apparent to others skilled in the art. It is the intent, therefore, to be limited only by the scope of the impending claims and not by the specific details presented by way of description and explanation above.

References:

[1] A. Vergara , E. Martinelli, E. Llobet, A. D'Amico, C. Di Natale, "Optimized Feature Extraction for Temperature-Modulated Gas Sensors", Journal of Sensors Volume 2009, Article ID 716316.

[2] Alexey Lipatov, Alexey Varezhnikov, Peter Wilson, Victor Sysoev, Andrei Kolmakov and Alexander Sinitskii, "Highly selective gas sensor arrays based on thermally reduced graphene oxide", Nanoscale, 2013, 5, 5426-5434.

[3] "The challenges with electrochemical NO2 sensors in outdoor air monitoring", hftps://www.aeroqual.com/challenges-electrochemical-no2-sensors-outdoor-ai r-monitoring.

[4] Gregory Koch, Richard Zemel, Ruslan Salakhutdinov Siamese Neural Networks for One-shot Image Recognition MAML or Meta learning computer vision/NLP, ICML deep learning workshop, 2015.

[5] Elad Hoffer, Nir Ailon, Deep metric learning using Triplet network, 2014, https://arxiv.org/abs/1412.6622v4.

**Claims**

1. A gas sensing device for sensing one or more gases in a mixture of gases; the gas sensing device (1) comprising:

   one or more chemo-resistive gas sensors (2), wherein each of the gas sensors (2) is configured for generating signal samples (SIG) corresponding to concentrations of the one or more gases in the mixture of gases;
   one or more heat sources (3), wherein the one or more heat sources (3) are controlled in such way that the gas sensors (2) are each heated according to one or more temperature profiles (FTP, STP);
   a preprocessing processor (4) configured for receiving the signal samples (SIG) from each of the gas sensors (2) and for preprocessing the received signal samples (SIG) in order to generate preprocessed signal samples (PSS) for each of the gas sensors (2);
   a feature extraction processor (5) configured for receiving the preprocessed signal samples (PSS) and for extracting a set of feature values (FV) from each of the received preprocessed signal samples (PSS) of the gas sensors (2) based on characteristics of the received preprocessed signal samples (PSS) of the gas sensors (2);
   a discriminative embedding network processor (6) configured for receiving the sets of feature values (FV) and for creating for each of the sets of feature values (FV) a set of embedded feature values (EV), wherein the discriminative embedding network processor (6) comprises a first trained model based algorithm processor (7) and a first trained model (8) for the first trained model based algorithm processor (7), wherein the first trained model (8) is configured for applying a loss function using discriminate weights to the sets of feature values (FV) in order to create the sets of embedded features values (EV);
   a classification processor (9) configured for receiving the sets of embedded feature values (EV) and for creating a classification value (CV) for each set of the embedded feature values (EV), wherein the classification value (CV) indicates a class of the mixture of gases, wherein the classification processor (9) comprises a second trained model based algorithm processor (10) and a second trained model (11) for the second trained model based algorithm processor (10), wherein the sets of embedded feature values (EV) are fed to an input (12) of the second trained model based algorithm processor (10), wherein the classification values (CV) are provided at an output (13) of the second trained model based algorithm processor (10); and
   a quantification processor (14) configured for receiving the sets of embedded feature values (EV) and the classification values (CV), wherein the quantification processor (14) is configured for creating for each of the gases a sensing result (SR) for each of the sets of embedded feature values (EV), wherein the quantification processor (14) comprises a third trained model based algorithm processor (15) and a plurality of third trained models (16) for the third trained model based algorithm processor (15), wherein the sets of embedded feature values (EV) are fed to an input (17) of the third trained model based algorithm processor (15), wherein the sensing result (SR) are provided at an output (18) of the third trained model based algorithm processor (15), wherein one third trained model (16) of the plurality of third trained models (16) is selected for creating the sensing results (SR) based on the classification values (CV).

2. A gas sensing device according to the preceding claim, wherein the one or more gas sensors (2) are alternately operated in recovery phases (RP) and in sense phases (SP);
wherein the one or more heat sources (3) are controlled in such way that the gas sensors (2) are each heated according to one or more first temperature profiles (FTP) of the one or more temperature profiles (FTP, STP) during the recovery phases (RP) and according to one or more second temperature profiles (STP) of the one or more temperature profiles (FTP, STP) during the sense phases (SP), wherein for each of the gas sensors (2) a maximum temperature of the respective first temperature profile (FTP) is higher than a maximum temperature of the respective second temperature profile (STP).

3. A gas sensing device according to one of the preceding claims, wherein a number of the chemo-resistive gas sensors (2) is greater than one, wherein at least some of the chemo-resistive gas sensors (2) have different sensitivities towards one or more of the gases.

4. A gas sensing device according to one of the preceding claims, wherein the preprocessing processor (4) is configured for executing a baseline calibration algorithm for the signal samples (SIG) received from the gas sensors (2).

5. A gas sensing device according to one of the preceding claims, wherein the preprocessing processor (4) is configured for executing a filtering algorithm for the signal samples (SIG) received from the gas sensors (2).

6. A gas sensing device according to one of the preceding claims, wherein the feature extraction processor (5) is configured for extracting from the received preprocessed signal samples (PSS) a normalized sensor sensitivity as one of the feature values (FV) for each of the gas sensors (2).

7. A gas sensing device according to one of the preceding claims, wherein the feature extraction processor (5) is configured for extracting from the received preprocessed signal samples (PSS) a slope of one of the preprocessed signal samples (PSS) as one of the feature values (FV) for each of the gas sensors (2).

8. A gas sensing device according to one of the preceding claims, wherein the feature extraction processor (5) is configured for extracting from the received preprocessed signal samples (PSS) for each of the gas sensors (2) a time correlation between a first of the preprocessed signal samples (PSS) of the respective gas sensor (2) and a second preprocessed signal sample (PSS) of the respective gas sensor (2) as one of the feature values (FV) for the respective gas sensor (2).

9. A gas sensing device according to one of the preceding claims, wherein the feature extraction processor (5) is configured for extracting from the received preprocessed signal samples (PSS) for each of the gas sensors (2) a spatial correlation between one of the preprocessed signal samples (PSS) of the respective gas sensor (2) and one of the preprocessed signal sample (PSS) of another of the gas sensors (2) as one of the feature values (FV) for the respective gas sensor (2).

10. A gas sensing device according to one of the preceding claims, wherein the first trained model based algorithm processor (7) is implemented as a first artificial neural network.

11. A gas sensing device according to one of the preceding claims, wherein the discriminative embedding network processor (6) comprises a plurality of first gated recurrent units (19) and a discriminative loss computation processor (20), which are configured for optimizing parameters, in particular weights and/or offsets, of the first trained model (8).

12. A gas sensing device according to one of the preceding claims, wherein the second trained model based algorithm processor (10) is implemented as a second artificial neural network, in particular as a fully connected artificial neural network.

13. A gas sensing device according to one of the claims 1 to 11, wherein the second trained model based algorithm

**EP 4 166 942 A1**

processor (10) is implemented as an incremental linear discriminant analysis processor.

14. A gas sensing device according to one of the preceding claims, wherein the third trained model based algorithm processor (15) is implemented as a third artificial neural network, in particular as a second gated recurrent unit (21) followed by a fully connected artificial neural network (22).

15. A gas sensing device according to one of the preceding claims, wherein the classification processor (9) is configured for preventing the quantification processor (14) from creating sensing results, in case the classification processor (9) is unable to create one of the classification value (CV) for one of the sets of the embedded feature values (EV).

16. A method for operating a gas sensing device for sensing one or more gases in a mixture of gases; the gas sensing device (1) comprising one or more chemo-resistive gas sensors (2), wherein the method comprises the steps of:

using each of the gas sensors (2) for generating signal samples (SIG) corresponding to concentrations of the one or more gases in the mixture of gases;
using one or more heat sources (3) for heating each of the gas sensors (2) according to one or more temperature profiles (FTP, STP);
using a preprocessing processor (4) for receiving the signal samples (SIG) from each of the gas sensors (2) and for preprocessing the received signal samples (SIG) in order to generate preprocessed signal samples (PSS) for each of the gas sensors (2);
using a feature extraction processor (5) for receiving the preprocessed signal samples (PSS) and for extracting one or more feature values (FV) from the received preprocessed signal samples (PSS) of each of the gas sensors (2) based on characteristics of the received preprocessed signal samples (PSS) of the respective gas sensor (2);
using a discriminative embedding network processor (6) for receiving the sets of feature values (FV) and for creating for each of the sets of feature values (FV) a set of embedded feature values (EV), wherein the discriminative embedding network processor (6) comprises a first trained model based algorithm processor (7) and a first trained model (8) for the first trained model based algorithm processor (7), wherein the first trained model (8) is configured for applying a loss function using discriminate weights to the sets of feature values (FV) in order to create the sets of embedded features values (EV);
using a classification processor (9) for receiving the sets of embedded feature values (EV) and for creating a classification value (CV) for each set of the embedded feature values (EV), wherein the classification value (CV) indicates a class of the mixture of gases, wherein the classification processor (9) comprises a second trained model based algorithm processor (10) and a second trained model (11) for the second trained model based algorithm processor (10), wherein the sets of embedded feature values (EV) are fed to an input (12) of the second trained model based algorithm processor (10), wherein the classification values (CV) are provided at an output (13) of the second trained model based algorithm processor (10); and
using a quantification processor (14) configured for receiving the sets of embedded feature values (EV), for receiving the classification values (CV), and for creating for each of the gases a sensing result (SR) for each of the sets of embedded feature values (EV), wherein the quantification processor (14) comprises a third trained model based algorithm processor (15) and a plurality of third trained models (16) for the third trained model based algorithm processor (15), wherein the sets of embedded feature values (EV) are fed to an input (17) of the third trained model based algorithm processor (15), wherein the sensing result (SR) are provided at an output (18) of the third trained model based algorithm processor (15), wherein one third trained model (16) of the plurality of third trained models (16) is selected for creating the sensing results (SR) based on the classification values (CV).

14

Figure 1

EP 4 166 942 A1

Figure 2

Figure 3

$$P_{cl}(t) = \{ \text{Gas1-only}, ..., \text{GasN-only}, ...\text{Mixture N}\}$$

9

13

CV

$F_{em}(t)$

FC

unknown gas

EV

12

10

$W_{cl}, b_{cl}$

11

Figure 4

Figure 5

Figure 6

Figure 7

Figure 8

Figure 9

EP 4 166 942 A1

Figure 10

EP 4 166 942 A1

Figure 11

Europäisches Patentamt
European Patent Office
Office européen des brevets

# EUROPEAN SEARCH REPORT

Application Number

EP 21 20 2164

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CHEN ZHIYUN ET AL: "Concentration Estimator of Mixed VOC Gases Using Sensor Array With Neural Networks and Decision Tree Learning", IEEE SENSORS JOURNAL, IEEE, USA, vol. 17, no. 6, 15 March 2017 (2017-03-15) , pages 1884-1892, XP011641631, ISSN: 1530-437X, DOI: 10.1109/JSEN.2017.2653400 [retrieved on 2017-02-17] * figures 1,3, 6 * * abstract * * section A. Metal Oxide Sensor Array section B. Sensing Measurements; page 1885 * * section B. Classification; page 1888 * * table II * * section: Step 2 Regression Based on Neural Networks; page 1887 * * page 1886, left-hand column, last line – page 1886, right-hand column, line 2 * | 1-16 | INV. G01N33/00 G01N27/12 |
| Y | CHU JIFENG ET AL: "Identification of gas mixtures via sensor array combining with neural networks", SENSORS AND ACTUATORS B: CHEMICAL, ELSEVIER BV, NL, vol. 329, 24 October 2020 (2020-10-24), XP086455395, ISSN: 0925-4005, DOI: 10.1016/J.SNB.2020.129090 [retrieved on 2020-10-24] * section 3.2 CNN-based gas identification; page 6 * | 1-16 | TECHNICAL FIELDS SEARCHED (IPC) G01N |

-/--

| | The present search report has been drawn up for all claims | | |
|---|---|---|---|

| Place of search | Date of completion of the search | Examiner |
|---|---|---|
| Munich | 15 March 2022 | Kratz, Dorothee |

EPO FORM 1503 03.82 (P04C01)

page 1 of 2

# EUROPEAN SEARCH REPORT

**Application Number**

EP 21 20 2164

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 736 564 A1 (INFINEON TECHNOLOGIES AG [DE]) 11 November 2020 (2020-11-11)<br>* figure 10 *<br>* paragraphs [0122], [0124] *<br>----- | 2 | |
| Y | R. GUTIERREZ-OSUNA: "Pattern analysis for machine olfaction: a review", IEEE SENSORS JOURNAL, vol. 2, no. 3, 7 November 2002 (2002-11-07), pages 189-202, XP055601218, USA ISSN: 1530-437X, DOI: 10.1109/JSEN.2002.800688<br>* figure 1 *<br>* table 1 *<br>* section C;<br>page 193, right-hand column *<br>----- | 3,4,10 | |
| | | | **TECHNICAL FIELDS SEARCHED (IPC)** |

The present search report has been drawn up for all claims

| Place of search | Date of completion of the search | Examiner |
|---|---|---|
| Munich | 15 March 2022 | Kratz, Dorothee |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 2164

This annex lists the patent family members relating to the patent documents cited in the above-mentioned European search report.
The members are as contained in the European Patent Office EDP file on
The European Patent Office is in no way liable for these particulars which are merely given for the purpose of information.

15-03-2022

| Patent document cited in search report | | Publication date | Patent family member(s) | | Publication date |
|---|---|---|---|---|---|
| EP 3736564 | A1 | 11-11-2020 | CN | 111914870 A | 10-11-2020 |
| | | | EP | 3736564 A1 | 11-11-2020 |
| | | | KR | 20200130657 A | 19-11-2020 |
| | | | US | 2020355662 A1 | 12-11-2020 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. VERGARA ; E. MARTINELLI ; E. LLOBET ; A. D'AMICO ; C. DI NATALE.** Optimized Feature Extraction for Temperature-Modulated Gas Sensors. *Journal of Sensors,* vol. 2009
- **ALEXEY LIPATOV ; ALEXEY VAREZHNIKOV ; PETER WILSON ; VICTOR SYSOEV ; ANDREI KOLMAKOV ; ALEXANDER SINITSKII.** Highly selective gas sensor arrays based on thermally reduced graphene oxide. *Nanoscale,* 2013, vol. 5, 5426-5434
- **GREGORY KOCH ; RICHARD ZEMEL ; RUSLAN SALAKHUTDINOV SIAMESE.** Neural Networks for One-shot Image Recognition MAML or Meta learning computer vision/NLP. *ICML deep learning workshop,* 2015
- **ELAD HOFFER ; NIR AILON.** *Deep metric learning using Triplet network,* 2014, https://arxiv.org/abs/1412.6622v4